# FASCICULE DE BREVET EUROPEEN

(11) **EP 2 503 336 B1**
(45) Date de publication et mention de la délivrance du brevet: **12.11.2014**
(21) Numéro de dépôt: 12160615.6
(22) Date de dépôt: 21.03.2012
(51) Int. Cl.: G01N 33/543, G01N 33/68, G01N 33/86

(54) **Nanobilles recouvertes de plasminogène comme support direct d'amplification cyclique de la protéine prion PrPsc**
Mit Plasminogen beschichtete Nanobeads als direkter Träger zur zyklischen Amplifikation des Prion-Proteins PrPsc
Nanobeads coated with plasminogen as direct support for cyclic amplification of prion protein PrPsc

(30) Priorité: 21.03.2011 FR 1152298; 21.03.2011 US 201161454727 P
(43) Date de publication de la demande: 26.09.2012
(73) Titulaire: Etablissement Français du Sang, 93210 La Plaine Saint Denis (FR)
(72) Inventeur: Segarra, Christiane, 34270 Claret (FR); Coste Van Der Luur, Joliette, 34000 Montpellier (FR); Bougard, Daisy, 34770 Gigean (FR)
(74) Mandataire: Domenego, Bertrand

(56) Documents cités:
- EP-A1- 1 435 521
- WO-A1-02/00713
- WO-A1-2007/101631
- M. B. MILLER ET AL: "Superparamagnetic Nanoparticle Capture of Prions for Amplification", JOURNAL OF VIROLOGY, vol. 85, no. 6, 15 mars 2011 (2011-03-15), pages 2813-2817, XP055011317, ISSN: 0022-538X, DOI: 10.1128/JVI.02451-10
- MAYS CHARLES E ET AL: "Plasminogen stimulates propagation of protease-resistant prion protein in vitro.", THE FASEB JOURNAL : OFFICIAL PUBLICATION OF THE FEDERATION OF AMERICAN SOCIETIES FOR EXPERIMENTAL BIOLOGY DEC 2010 LNKD- PUBMED:20732953, vol. 24, no. 12, décembre 2010 (2010-12), pages 5102-5112, XP009153686, ISSN: 1530-6860
- MASSIMILIANO CUCCIOLONI ET AL: "Binding of recombinant PrPc to human plasminogen: Kinetic and thermodynamic study using a resonant mirror biosensor", PROTEINS: STRUCTURE, FUNCTION, AND BIOINFORMATICS, vol. 58, no. 3, 15 février 2005 (2005-02-15), pages 728-734, XP055011316, ISSN: 0887-3585, DOI: 10.1002/prot.20346

## Description

La présente invention concerne un procédé in vitro de détection d'un conformère pathogène de la protéine prion dans un échantillon, ledit procédé comprenant une étape préalable de capture du conformère pathogène par mise en contact de l'échantillon avec des nanobilles recouvertes d'un ligand du conformère pathogène, puis la mise en oeuvre d'une amplification cyclique de la protéine prion mal repliée directement sur le support solide ayant capturé le conformère pathogène, et la détection de la présence de conformère pathogène. L'invention concerne également l'utilisation d'un kit pour la mise en oeuvre de ce procédé.

Les maladies conformationelles constituent un groupe de maladies non apparentées les unes aux autres mais qui partagent les mêmes mécanismes moléculaires impliquant la transition conformationelle d'une protéine existante (conformère non pathogène) vers un repliement aberrant (conformère pathogène) conduisant à des agrégations protéiques et la formation de dépôts tissulaires.

Des exemples typiques de ces maladies sont les encéphalopathies spongiformes transmissibles et la maladie d'Alzheimer. Les encéphalopathies spongiformes transmissibles sont relativement rares chez les humains mais ont fait l'objet d'une attention particulière lorsque les risques de transmission de la forme bovine de l'encéphalopathie spongiforme à l'homme, à travers la chaîne alimentaire, ont été révélés. La variante de la maladie de Creutzfeldt-Jacob (vMCJ) est la contrepartie humaine de l'encéphalopathie spongiforme bovine.

Au début de l'année 2010, 246 cas primaires de vMCJ avaient été répertoriés dans 11 pays différents. Bien que l'incidence clinique de la vMCJ reste faible, la prévalence globale de la maladie au stade préclinique est évaluée à 1:4000 au Royaume-Uni. La protéine prion cellulaire normale (PrP^{c}), en subissant une transition conformationelle vers un conformère pathogène anormalement replié (PrP^{SC}), est à l'origine des encéphalopathies spongiformes transmissibles. La protéine PrP^{SC} est principalement trouvée au niveau du cerveau et des organes lymphoïdes des animaux infectés, mais elle peut aussi être détectée dans de nombreux tissus incluant le muscle squelettique, le foie, le pancréas, les reins, l'utérus, et la peau.

Cette distribution périphérique de la PrP^{SC} pose le problème de la transmission potentielle inter-individus de la variante de la MCJ, par chirurgie ou procédure médicale invasive, telle que la transfusion sanguine ou les transplantations.

Il est donc particulièrement important de pouvoir détecter de manière sensible et spécifique les dons de sang qui auront pu être récoltés à partir de patients atteints de la variante de la maladie de Creutzfeldt-Jacob. Le contrôle sanitaire des aliments d'origine animale (lait, viandes) est également important.

Plus généralement, le développement d'une méthode hautement sensible et spécifique pour détecter des conformères pathogènes de maladies à prion présente une utilité pour le diagnostic de ces maladies chez des patients, en particulier lorsqu'ils sont encore dans une phase asymptomatique.

La capacité de la protéine prion PrP^{SC} de catalyser la transformation du conformère non pathogène en un conformère pathogène a été mise à profit pour développer une méthode de détection des conformères pathogènes par amplification cyclique. Cette technologie, appelée "amplification cyclique des protéines mal repliées" (PMCA) a été initialement décrite par Saborio et al. (Nature, 2001,401 (6839):810-3). L'amplification cyclique repose sur la conversion d'un substrat constitué de conformères non pathogènes par des quantités non détectables de conformères pathogènes, de manière à obtenir des niveaux de conformères pathogènes détectables par des méthodes conventionnelles telles que les immunoblots. Ceci est rendu possible par la répétition de cycles d'incubation du conformère pathogène en présence du conformère non pathogène, permettant d'accroître la quantité de conformère pathogène, suivie de la désagrégation des agrégats formés, de manière à rendre accessible les conformères pathogènes pour catalyser la conversion de nouveaux conformères non pathogènes.

Cependant, l'amplification cyclique de la protéine PrP^{SC} directement à partir du sang, reste difficile, notamment en raison des grands volumes d'échantillons requis pour permettre une détection et en raison de la présence dans le sang d'inhibiteurs de la protéine PrP^{SC}.

Pour remédier à ces problèmes, les inventeurs ont cherché à développer une méthodologie de PMCA améliorée, avec une étape préalable de concentration de la protéine PrP^{SC}.

Des essais menés utilisant le phosphotungstate de sodium ou le sulfate de streptomycine, s'ils ont effectivement conduit à une concentration de la protéine PrP^{SC}, n'ont pas permis ensuite une amplification efficace du conformère pathogène PrP^{SC} par PMCA.

Les inventeurs sont parvenus à développer une méthode extrêmement sensible de détection de la protéine PrP^{SC}, basée sur la capture du conformère pathogène de la protéine prion présent dans l'échantillon à analyser, à l'aide d'un support solide constitué de nanobilles recouvertes de plasminogène, suivie de l'amplification directe du conformère pathogène lié au support solide par PMCA, puis détection des conformères pathogènes amplifiés.

Il a récemment été décrit que des nanoparticules superparamagnétiques d'oxyde de fer avaient la capacité de lier sélectivement la protéine PrP^{SC}.(Miller et Supattapone, J. Virol. 2011 Vol.85, No.6, p.2813-2817 [Epub ahead of print Jan 12.]). La capacité de la protéine PrP^{SC} à lier les nanoparticules a été mise en évidence en mettant en oeuvre un cycle d'amplification PMCA directement sur les nanoparticules ayant lié PrP^{SC}. Cet article a suggéré que la mise en oeuvre d'une étape de concentration par liaison de PrP^{SC} avec les nanoparticules magnétiques, couplée à une PMCA, pourrait améliorer la détection de faibles concentrations de protéines prions. Les niveaux d'amplification montrés après 72 heures de PMCA restent cependant très faibles, 5 µl de cerveau à 5% ne donnant pas de signal sans PMCA et un signal très faible avec PMCA pour la souche RML des souris CD1, et un faible signal pour la souche 237 de hamster.

Il a par ailleurs été rapporté que le plasminogène agit comme un cofacteur stimulant la conversion de la protéine PrP^{C}, la vitesse de production de PrP^{SC} étant multipliée par environ 2,5 en présence de 0,5 µM de plasminogène humain dans le milieu réactionnel de PMCA (Mays et Ryou, Prion. 2011;5(1):22-7 ; Mays et Ryou, FASEB J. 2010;24(12):5102-12).

Le principe de la mise en oeuvre d'une amplification PMCA après concentration de la protéine PrP^{SC} à l'aide de billes recouvertes d'un ligand de la protéine PrP^{SC} a été décrite par les inventeurs (Segarra et al., Transfusion Clinique et Biologique 2009, 16(3) : 295), sans toutefois identifier la nature du ligand utilisé.

Or plusieurs types de ligand de la protéine PrP^{SC} ont été identifiés et incluent notamment des acides nucléiques aptamères (Sayer et al., J. Biol. Chem. 2004, 279(13):13102-13109), des anticorps anti-ADN (Zou et al., Proc. Natl. Acad. Sci. USA, 2004, 101:1380-1385), des anticorps monoclonaux anti-PrP^{SC}, (Korth et al., Nature 1997, 390 : 74-77, et Paramithiotis et al., Nat. Med. 2003, 9 : 893-899) et des ligands polyanioniques (demande de brevet internationale WO 03/073106).

Les inventeurs ont ainsi démontré, de manière inattendue, que le plasminogène lié à des nanobilles permet de lier les conformères pathogènes PrP^{SC} et que ceux-ci restent accessibles pour la conversion de conformères non pathogènes PrP^{C} en conformères pathogènes PrP^{SC}.

La spécificité du plasminogène pour la protéine PrP^{SC} est controversée, le plasminogène ayant à la fois été rapporté lier exclusivement la protéine PrP^{SC} (voir par exemple Fischer et al. Nature 2000, 408(6811) :479-483 ; Maissen et al., The Lancet, 2001, 357(9273) :20262028)) alors que d'autres travaux ont montré que le plasminogène liait également la protéine PrP^{C} (Cuccioloni et al., Proteins 2005 ; 58(3) :728-734).

Ainsi, la demande WO 02/00713 décrit que le plasminogène lie PrP^{SC} et qu'il peut être employé dans une méthode de concentration de PrP^{SC} par traitement d'un échantillon fluide avec une phase solide portant du plasminogène.

La demande EP1435521 décrit que le plasminogène permet de différencier PrP^{SC} de PrP^{C} et que le plasminogène lié à des billes magnétiques permet de précipiter sélectivement PrP^{SC}.

Sans avoir clarifié si les billes recouvertes de plasminogène capturaient seulement la protéine PrP^{SC} ou également les isoformes PrP normal à partir des échantillons à analyser, les inventeurs ont fait l'hypothèse que la présence de PrP^{C} résiduelle en plus de la protéine du conformère pathogène PrP^{SC} ne représenterait pas un problème majeur compte tenu du fait que l'amplification cyclique des protéines mal repliées réalisée ensuite utilise la protéine PrP^{C} en tant que substrat pour l'amplification de la protéine PrP^{SC}.

La mise en oeuvre du procédé des inventeurs sur des plasmas dopés par des homogénats de cerveau infecté de souris transgéniques modèles de la tremblante du mouton (lignée tg 338) a permis la détection de la protéine PrP^{SC} dans des dilutions d'un facteur 10⁻¹⁰ d'homogénats de cerveaux infectés, correspondant à l'équivalent d'une quantité initiale de 10 pg de cerveau. Une sensibilité plus élevée que celle obtenue par un bioessai effectué sur la même souche a ainsi été obtenu (dilution 10⁻⁸ ; Tixador et al., PLoS Pathog 2010, 6(4:e1000859)). La sensibilité du procédé ainsi développé correspondrait à moins de 0,01 fois la dose létale moyenne. Le procédé des inventeurs appliqué à la détection de la protéine prion dans des globules blancs de mouton, est sensible à 100%.

Par ailleurs, il a été montré que l'étape de capture est essentielle, lorsque le procédé est mis en oeuvre sur des échantillons de sang, pour pouvoir détecter la protéine PrP^{SC} dans les échantillons infectieux, la PMCA réalisée sans capture préalable conduisant à des résultats faux-négatifs.

Le procédé appliqué à la détection de la variante de la maladie de Creutzfeldt-Jacob humaine a permis la détection de la protéine PrP^{SC} humaine dans des échantillons de plasma humain additionné d'homogénats de cerveaux infectés avec l'équivalent d'une quantité initiale aussi faible que 100 pg de cerveau.

Par rapport à la méthodologie d'amplification cyclique de protéines mal repliées décrite par Jones et al. (Transfusion 2009, 49(2):376-84) qui détectait la protéine PrP^{SC} en utilisant les plaquettes en tant que substrat d'amplification, le procédé selon la présente demande a permis d'obtenir une sensibilité 100 fois plus élevée.

Le procédé in vitro de détection de conformère pathogène d'une protéine caractéristique d'une maladie conformationelle décrit dans la présente demande constitue donc une amélioration indéniable vis-à-vis des méthodes de détection aujourd'hui disponibles.

### Définitions

Le terme « maladie conformationelle » désigne un ensemble de maladies caractérisées par des phénomènes d'agrégation protéique et de dépôts tissulaires liés à la transition conformationelle d'une protéine, caractéristique de la maladie conformationelle, entre un conformère normal, non pathogène, et un conformère pathogène.

Les maladies conformationelles incluent notamment les encéphalopathies spongiformes transmissibles.

L'encéphalopathie spongiforme transmissible (EST) désigne les formes humaines d'EST, en particulier la maladie de Creutzfeldt-Jacob (MCJ) ou sa variante (vMCJ), la maladie de kuru, la maladie de Gerstmann-Straussler-Scheiker (GSS) et l'insomnie familiale fatale (IFF), et les formes animales d'EST, en particulier la tremblante du mouton (ou « scrapie »), l'encéphalopathie spongiforme bovine (ESB), l'encéphalopathie transmissible du vison (ETV) et la maladie du dépérissement chronique (MDC) du cervidé. Une encéphalopathie spongiforme transmissible est de préférence la MCJ ou sa variante, de préférence encore la vMCJ.

Par « protéine caractéristique d'une maladie conformationelle », on entend une protéine qui existe normalement sous la forme d'un conformère non pathogène chez un individu sain et qui peut subir une transition conformationelle conduisant à un repliement anormal (conformère pathogène). Le conformère pathogène est généralement insoluble et forme des agrégats. Le conformère pathogène a la capacité d'interagir spécifiquement avec le conformère non pathogène et de catalyser sa conversion en conformère pathogène, assurant ainsi une propagation de la transition conformationelle du conformère pathogène au conformère non pathogène.

La protéine caractéristique des encéphalopathies spongiformes transmissibles est la protéine prion (PrP) dont le conformère non pathogène est généralement désigné Prp^{C} et le conformère pathogène PrP^{SC} ou PrP^{EST}.

### Procédé de détection d'un conformère pathogène de la protéine prion

L'invention concerne un procédé de détection d'un conformère pathogène de la protéine prion qui comporte une étape de concentration du conformère anormal par capture du conformère anormal présent dans un échantillon à analyser à l'aide d'un ligand du conformère anormal, le plasminogène, fixé sur un support solide constitué de nanobilles, une étape d'amplification cyclique de protéines prions mal repliées étant ensuite directement mise en oeuvre sur le support solide ayant fixé des molécules de conformère anormal.

L'invention est donc relative à un procédé in vitro de détection d'un conformère pathogène de la protéine prion (PrP^{SC}) dans un échantillon, ledit procédé comprenant les étapes consistant à :
a) mettre en contact des nanobilles recouvertes de plasminogène avec un échantillon;
b) séparer lesdites nanobilles recouvertes de plasminogène de l'échantillon ;
c1) mettre en contact lesdites nanobilles recouvertes de plasminogène obtenues à l'étape b) avec une préparation comprenant du conformère non pathogène de la protéine prion (PrP^{C}) ;
c2) désagréger les agrégats éventuellement formés durant l'étape c1) ;
d) détecter la présence de PrP^{SC} dans la préparation, la présence de PrP^{SC} dans la préparation étant indicative de la présence de PrP^{SC} dans l'échantillon ;
les étapes c1) et c2) formant un cycle qui est répété au moins deux fois avant de mettre en oeuvre l'étape d).

Un échantillon à analyser au sens de l'invention peut être tout fluide ou solide alimentaire, en particulier d'origine animale, tel que le lait, la viande, et tout échantillon biologique d'un sujet humain ou animal.

Le terme « sujet » désigne un mammifère humain ou non humain, tel qu'un bovin, un ovin, un porcin, un cervidé, un félin, un canin, un rongeur, un vison.

Un « échantillon biologique » peut désigner un fluide biologique, des cellules ou un tissu, comme par exemple le sang, la lymphe, l'urine, le lait, le tissu cérébral, la moelle épinière, des tissus ou cellules lymphoïdes, ou tout produit dérivé d'une source humaine ou animale susceptible d'être contaminé par un conformère pathogène tel que, par exemple, une préparation d'hormone de croissance ou un extrait tissulaire. Un échantillon biologique peut désigner également un prélèvement de sang, de plasma, ou un organe destiné à la transplantation.

Lorsque l'échantillon est un tissu, le procédé selon l'invention peut être mis en oeuvre sur un homogénat du tissu.

Par « nanobilles » on désigne des billes d'un diamètre compris entre 1 et 900 nm, de préférence entre 10 et 750 nm, de préférence encore entre 30 et 500 nm, de préférence encore entre 50 et 300 nm, ou encore entre 75 et 150 nm.

Les nanobilles sont de préférence paramagnétiques. Les nanobilles paramagnétiques contiennent généralement un pourcentage élevé d'oxydes de fer, par exemple au moins 50% en poids, ou encore au moins 70% en poids. Des nanobilles paramagnétiques disponibles dans le commerce, comme par exemple celles distribuées par Ademtech (France), peuvent être utilisées pour la mise en oeuvre de l'invention. Il peut s'agir en particulier de nanobilles carboxyliques superparamagnétiques telle que les billes Carboxyl-Adembeads de la société Ademtech.

Sans vouloir être liés par cette théorie, les inventeurs pensent que l'utilisation de nanobilles paramagnétiques est importante pour la mise en oeuvre de la méthode selon l'invention, car les nanobilles, en se repoussant constamment du fait de leur paramagnétisme, resteraient plus longtemps en suspension, favorisant ainsi l'accessibilité des protéines PrP^{SC} captées aux protéines PrP^{C} du mélange réactionnel de PMCA. En effet, des essais menés avec des microbilles n'ont pas permis d'obtenir une amplification satisfaisante des protéines PrP^{SC} captées lorsque les étapes d'incubation sont effectuées en l'absence d'agitation.

Les nanobilles sont recouvertes de plasminogène, et éventuellement d'au moins un autre type de ligand du conformère pathogène de la protéine prion.

Le plasminogène est de préférence le plasminogène humain.

Un « ligand d'un conformère pathogène de la protéine prion » est une molécule qui peut être de toute nature, par exemple peptidique, protéique, acide nucléique, lipidique, ionique, etc., et qui est capable de fixer un conformère pathogène de la protéine prion. Le ligand peut lier le conformère pathogène préférentiellement au conformère non pathogène, de préférence encore il peut lier spécifiquement le conformère pathogène. Le fait que le ligand puisse lier à la fois le conformère pathogène et le conformère non pathogène n'est pas limitant pour la mise en oeuvre de l'invention puisque l'étape de capture du conformère pathogène est ensuite suivie d'une amplification cyclique de protéines mal repliées au cours de laquelle le conformère non pathogène constitue le substrat d'amplification. Une liaison préférentielle ou spécifique du ligand au conformère pathogène, par rapport au conformère non pathogène, est toutefois préférable de manière à éviter de saturer le support solide avec une partie de conformère non pathogène, avec le risque de réduire l'efficacité de la capture du conformère pathogène à partir de l'échantillon à analyser.

Un ligand du conformère pathogène PrP^{SC} peut être sélectionné dans le groupe constitué des acides nucléiques aptamères (voir Sayer et al., J. Biol. Chem. 2004, 279(13):13102-13109), des anticorps anti-ADN (voir Zou et al., Proc. Natl. Acad. Sci. USA, 2004, 101:1380-1385), des anticorps monoclonaux anti-PrP^{SC}, (voir par exemple Korth et al., Nature 1997, 390 : 74-77, et Paramithiotis et al., Nat. Med. 2003, 9 : 893-899) et des ligands polyanioniques.

Des ligands polyanioniques appropriés ont été décrits dans la demande de brevet internationale WO 03/073106, et incluent notamment les polyglycosides polyanioniques, en particulier les polyglycosides polysulfonés, de préférence le polysulfate de pentosan ou le sulfate de dextran ; les polyéthylèneimines ; les polyamines, notamment les polylysines ; les polyamidoamines, par exemple les dendrimères poly(amidoamine) ; et les polyamines quaternaires telles que le poly(chlorure de diallyldimethylammonium) ou le polybrène (ou 1,5-diméthyl-1,5-diazaudécaméthylène polyméthobromure ou bromure d'héxadiméthrine). Ce type de ligands a été décrit lier les protéines PrP^{SC}, β-amyloïde et tau.

Les aptamères constituent une classe de molécules qui représentent une alternative aux anticorps en terme de reconnaissance moléculaire. Les aptamères sont des séquences d'acides nucléiques ayant la capacité de reconnaître virtuellement n'importe quelle classe de molécules cibles avec une haute affinité et spécificité. De tels ligands peuvent être isolés par un criblage appelé SELEX (Systematic Evolution of Ligands by EXponential enrichment) d'une banque de séquences aléatoires, comme décrit dans Tuerk et Gold (Science. 1990 3;249(4968):505-10). La banque de séquence aléatoire peut être obtenue par synthèse d'ADN par chimie combinatoire. Dans une telle banque, chaque membre est un oligomère linéaire, éventuellement chimiquement modifié, correspondant à une séquence unique. Les possibles modifications, applications et avantages de cette classe de molécules ont fait l'objet d'une revue par Jayasena (Clin Chem. 1999 45(9):1628-50). Les aptamères peuvent également être de nature peptidique. Ils consistent en une région variable conformationnellement contrainte, assemblée sur une plateforme protéique, telle que la Thioredoxin A de E. coli, et peuvent être sélectionnés à partir de banques combinatoires par des méthodes de double hybride (Colas et al., Nature1996, 380, 548-50.).

Le plasminogène, et éventuellement le ligand du conformère pathogène de la protéine prion peut être fixé sur les nanobilles paramagnétiques par tout moyen connu de l'homme du métier, par liaison directe ou indirecte, par exemple par moyen physique (chaleur, pression), ou chimique par création de liaisons covalentes ou non covalentes (hydrophobes, anioniques) par l'intermédiaire de groupement réactifs de type carboxyle, amine, ou passivement, par exemple si le ligand est un matériel polyanionique et que le support solide est composé de polystyrène, etc.

Dans l'étape a) du procédé selon l'invention, les nanobilles recouvertes de plasminogène sont mises en contact avec l'échantillon à analyser. Les nanobilles sont incubées au contact de l'échantillon pendant une durée suffisante et dans des conditions permettant la liaison au plasminogène de tout ou partie des conformères pathogènes PrP^{SC} présents dans l'échantillon.

La durée et les conditions d'incubation peuvent être variables en fonction de la nature de l'échantillon. Les conditions et la durée d'incubation seront déterminées de préférence de manière à ce que le plasminogène fixé sur les nanobilles puisse fixer un maximum de conformère pathogène PrP^{SC}.

L'incubation peut être effectuée à n'importe quelle température, par exemple au froid (4-10°C), à température ambiante ou à 37°C. Elle est préférentiellement mise en oeuvre à 37°C. L'incubation peut être effectuée avec agitation (par exemple de 1 rpm à 1400 rpm) ou sans agitation. L'incubation peut être effectuée pendant une durée de 1 minute à 5 jours, de préférence pendant 5 minutes à 24 heures, de préférence 10 minutes à 4 heures, de préférence 30 minutes à 3 heures, de préférence encore de 1 heure à 2 heures. La durée d'incubation est susceptible d'être ajustée en fonction des quantités respectives de conformère pathogène de la protéine prion dans l'échantillon et de plasminogène fixé sur le support solide.

Une optimisation des paramètres de capture a permis d'atteindre une efficacité de capture d'un rendement supérieur à 95%. Pour cela des essais croisés ont été réalisés entre la quantité de billes à utiliser pour la capture dans 500 µl de plasma et la concentration de plasminogène nécessaire à la fonctionnalisation des nanobilles. Les essais conduits sur une large gamme des deux composants ont permis d'observer que les quantités de nanobilles et la concentration de plasminogène devaient être définis de manière très fine pour une capture optimale. En effet contrairement aux idées reçues, les quantités maximales ne sont pas les plus efficaces. Ainsi, dans le cas de nanobilles carboxyliques paramagnétiques (Ademtech, France) l'amplification optimale est obtenue quand les billes sont fonctionnalisées avec une quantité de plasminogène correspondant à la quantité minimale de protéine conseillée par le fournisseur de nanobilles.

Ainsi, de préférence, les nanobilles, en particulier les nanobilles paramagnétiques, utilisées dans le cadre de l'invention, sont recouvertes de plasminogène à raison de 10 à 30 µg de plasminogène par mg de nanobilles, de manière davantage préférée à raison de 10 à 20 µg de plasminogène par mg de nanobilles, de manière préférée entre toutes à raison de 10 µg de plasminogène par mg de nanobilles.

De même pour les volumes de nanobilles carboxyliques paramagnétiques fonctionnalisées par le plasminogène utilisées pour la capture, le signal optimum est obtenu avec 10 µl de nanobilles à 1% (soit 10 µl d'une suspension de nanobilles à 1 mg de nanobilles par ml de suspension). Une décroissance de signal est observée quand on utilise 20 µl et enfin le signal disparaît lorsque la capture est réalisée avec 30 µl des nanobilles fonctionnalisées par le plasminogène. De même lorsque des quantités décroissantes de nanobilles fonctionnalisées par le plasminogène sont utilisées, le signal diminue de plus en plus lorsque 5 µl de nanobilles sont utilisés pour la capture puis à 2,5 µl de nanobilles.

Ainsi, de préférence, les nanobilles recouvertes de plasminogène sont mises en contact, à l'étape a) du procédé selon l'invention, avec un échantillon à un ratio de : 2,5 à 90 µl d'une suspension de nanobilles à 1% (poids/volume) pour 50 à 500 µl d'échantillon; de manière davantage préférée un ratio de:5 à 50 µl d'une suspension de nanobilles à 1 % (poids/volume) pour 250 à 500 µl d'échantillon; de manière préférée entre toutes un ratio de: 8 à 12 µl d'une suspension de nanobilles à 1% (poids/volume) pour 450 à 500 µl d'échantillon.

Ainsi, une fixation optimale du conformère pathogène PrP^{SC} sur des nanobilles recouvertes de plasminogène peut être réalisée par exemple en mettant 500 µl d'échantillon de plasma au contact de 10 µl d'une suspension de nanobilles (en particulier des nanobilles carboxyliques paramagnétique) à 1% (poids/volume) recouvertes à raison de 10 µg de plasminogène /mg de nanobilles, et incubés à 37°C pendant deux heures.

A l'issue de l'étape a), les nanobilles recouvertes de plasminogène sont séparées de l'échantillon (étape b)). Cette étape peut être mise en oeuvre en récoltant les nanobilles par aimantation ou par centrifugation. Avantageusement, les billes récoltées peuvent être lavées.

Les nanobilles ainsi récoltées après séparation de l'échantillon porteront des molécules de conformère pathogène PrP^{SC} liées au plasminogène, si l'échantillon analysé comprenait des protéines PrP^{SC}.

Il peut être utilisé immédiatement pour procéder à l'amplification cyclique des protéines prions mal repliées ou être stocké au froid, par exemple à 4°C, avant utilisation.

### Amplification cyclique des protéines prions mal repliées

L'amplification cyclique des protéines prions mal repliées est mise en oeuvre en utilisant directement le conformère pathogène PrP^{SC} lié aux nanobilles par l'intermédiaire de son ligand, le plasminogène.

Cette amplification est mise en oeuvre en répétant de manière cyclique les étapes consistant à :
c1) mettre en contact lesdites nanobilles obtenues à l'étape b) avec une préparation comprenant ledit conformère non pathogène PrP^{C} ; et
c2) désagréger les agrégats éventuellement formés durant l'étape c1).

La technologie d'amplification cyclique des protéines mal repliées est connue de l'homme du métier et a été décrite notamment dans la demande de brevet internationale WO 2002/004954.

La « préparation comprenant ledit conformère non pathogène PrP^{C} » peut être par exemple un homogénat de cerveau ou de tissu lymphoïde, ou un fluide biologique, tel que du sang complet, plasma, etc., ou un lysat cellulaire d'un individu sain, de préférence en provenance d'un individu de la même espèce que l'individu dont provient l'échantillon biologique à analyser (par exemple un homogénat de cerveau humain sain si un échantillon biologique humain est analysé). La préparation comprenant ledit conformère non pathogène PrP^{C} peut aussi être une solution comprenant du conformère non pathogène PrP^{C} extrait et éventuellement purifié, ou synthétique ou recombinant.

Avantageusement, le conformère non pathogène PrP^{C} utilisé peut être marqué, de manière radioactive ou fluorescente, en vue de l'étape de détection ultérieure.

De préférence, dans l'étape c1), la quantité de conformère non pathogène PrP^{C} utilisée est en excès par rapport à la quantité de conformère pathogène PrP^{SC} fixée aux nanobilles .

Généralement, le ratio initial de conformère non pathogène PrP^{C} par rapport au conformère pathogène PrP^{SC}, s'il est présent, lié aux nanobilles, sera supérieur à 100:1, de préférence supérieur à 1000:1, et de préférence encore supérieur à 1 000 000:1.

Les étapes c1) et c2) sont préférentiellement mises en oeuvre dans des conditions physiologiques (pH, température, et force ionique), éventuellement en présence d'inhibiteurs de protéases et de détergent. Les conditions sont préférentiellement choisies de manière à permettre aux conformères pathogènes PrP^{SC}, liés aux nanobilles par l'intermédiaire du plasminogène, de convertir les conformères non pathogènes PrP^{C},en conformères pathogènes PrP^{SC}, formant ainsi des agrégats ou des oligomères de conformères pathogènes PrP^{SC},.

Les conditions précises d'incubation et la durée d'incubation pourront être ajustées par l'homme du métier.

Par exemple, l'étape c1) peut être mise en oeuvre pendant 1 minute à 5 jours, de préférence pendant 1 minute à 24 heures, de préférence pendant 5 minutes à 6 heures, de préférence encore pendant 10 minutes à 4 heures, de préférence encore pendant 20 minutes à 2 heures, de préférence encore pendant 30 à 90 minutes, de préférence encore pendant 30 à 60 minutes. L'incubation peut être effectuée à n'importe quelle température, par exemple au froid (4-10°C), à température ambiante ou à 37°C. Elle est préférentiellement mise en oeuvre à 37°C. L'incubation peut être effectuée avec agitation (par exemple de 1 rpm à 1400 rpm) ou sans agitation.

L'étape c2) de désagrégation peut être effectuée par tous moyens connus de l'homme du métier, tels que le traitement avec des solvants (en présence de dodécylsulfate de sodium (SDS), acétonitrile, diméthylsulfoxide, urée, acide préfluoroacétyle dilué, acide formyle dilué, etc), la modification des paramètres physicochimiques de la préparation, tels que pH, température, force ionique, constante diélectrique, et les méthodes physiques, telles que sonication, irradiation laser, congélation, décongélation, presse française, incubation à l'autoclave, haute pression, mélange, etc. Préférentiellement, la désagrégation est mise en oeuvre par sonication.

L'étape c2) est effectuée de manière à ce que au moins une partie des agrégats éventuellement formés durant l'étape c1) soit désagrégée. La durée de l'étape c2) pourra être déterminée par l'homme du métier en fonction du moyen de désagrégation utilisé. L'étape c2) durera généralement entre 1 seconde et 1 heure. Une durée supérieure peut toutefois être envisagée. Si la désagrégation est effectuée par sonication, la durée de l'étape c2) pourra être, par exemple, de 1 seconde à 10 minutes, de préférence de 2 secondes à 5 minutes, de préférence de 5 secondes à 2 minutes, de préférence de 10 secondes à 1 minute, de préférence encore de 20 à 40 secondes.

La succession des étapes c1) et c2) constitue un cycle. Pour la mise en oeuvre du procédé selon l'invention, les étapes c1) et c2) sont mises en oeuvre successivement au moins deux fois, c'est-à-dire que le procédé comprend la répétition d'au moins deux cycles.

Le nombre de cycles à effectuer peut être déterminé par l'homme du métier de manière à obtenir une amplification suffisante du conformère pathogène PrP^{SC}, pour permettre sa détection ultérieure.

Le nombre de cycles mis en oeuvre dans le procédé concernant l'invention peut être compris par exemple entre 2 et 1000, de préférence entre 20 et 500, de préférence encore entre 50 et 350, de préférence encore entre 80 et 240.

En particulier, l'étape c1) peut être mise en oeuvre pendant 20 minutes à 2 heures, par exemple pendant 30 à 120 minutes, ou entre 30 et 90 minutes, ou encore entre 30 et 60 minutes, l'étape c2) mise en oeuvre par sonication pendant 10 secondes à 1 minute, par exemple pendant 20 à 40 secondes et le nombre de cycles peut être compris entre 50 et 350, par exemple entre 50 et 100 par tour d'amplification. La désagrégation peut être effectuée préférentiellement à l'aide d'un sonicateur Misonix 4000, avec une puissance comprise entre 60 et 80 %.

Les conditions optimales d'amplification dans des échantillons de plasma dopé d'homogénats de cerveau ont été identifiées comme 30 minutes d'incubation, 20 secondes de sonication à 80 % de puissance et 80 cycles, cette amplification pouvant être répétée si la quantité de conformère pathogène obtenue est trop faible pour permettre une détection sensible.

Très classiquement, l'amplification de la protéine PrP^{SC} par PMCA met en oeuvre des cycles dans lesquels l'étape c1) dure 30 minutes.

Les inventeurs ont toutefois réalisé que si, dans le tout premier cycle d'amplication par PMCA, la durée d'incubation de l'étape c1) est de 30 minutes seulement pour des échantillons à analyser extrêmement peu concentrés en protéine PrP^{SC}, la conversion de protéine PrP^{C} présente dans le mélange réactionnel de PMCA (c'est-à-dire ladite préparation comprenant le conformère non pathogène PrP^{C}) peut ne pas être suffisante pour amorcer efficacement l'amplification par PMCA. Dans ce cas, après l'étape c2) de désagrégation, il est possible qu'il ne subsiste pas assez de protéine PrP^{SC} apte à assurer la conversion conformationelle de la protéine PrP^{C}, et que, au final, la protéine PrP^{SC} présente dans l'échantillon ne puisse être détectée. L'allongement de la durée de l'étape c1) du tout premier cycle d'amplication, par exemple à environ 90 minutes, permet d'écarter le risque de résultat faux négatif.

Ainsi avantageusement la méthode selon l'invention comprend :
- un premier cycle d'amplification dans lequel l'étape c1) est mise en oeuvre pendant 60 à 120 minutes, de préférence pendant 80 à 100 minutes, de préférence encore pendant environ 90 minutes, l'étape c2) pouvant être effectuée dans les conditions telles que décrites ci-dessus, de préférence par sonication pendant 10 secondes à 1 minute, plus préférablement pendant 20 à 40 secondes, puis
- un ou plusieurs cycles d'amplification dans lesquels les conditions de mises en oeuvre des étapes c1) et c2) sont telles que décrites ci-dessus. Par exemple, l'étape c1) peut être mise en oeuvre pendant 30 à 60 minutes et/ou l'étape c2) peut être mise en oeuvre par sonication pendant 20 à 40 secondes. Le nombre de ces cycles peut être compris entre 49 et 99 par tour d'amplification. La désagrégation peut être effectuée préférentiellement à l'aide d'un sonicateur Misonix 4000, avec une puissance comprise entre 60 et 80 %.

A l'issue de l'amplification cyclique, la présence ou la quantité de conformère pathogène présent dans la préparation est détectée.

### Détection

La détection du conformère pathogène PrP^{SC} peut être précédée d'une étape d1) de destruction ou d'élimination du conformère non pathogène PrP^{C}, encore présent dans la préparation à l'issue de l'étape c2). Les conformères pathogènes PrP^{SC} étant généralement insolubles, même en présence de détergent non dénaturant, et étant résistants aux protéases, cette étape peut être réalisée typiquement par traitement avec une ou plusieurs protéases, telles que la protéinase K, la trypsine, etc., ou par centrifugation pour séparer les conformères pathogènes PrP^{SC} insolubles des conformères non pathogènes solubles.

La détection de conformère pathogène PrP^{SC} présent dans la préparation, éventuellement traitée comme indiqué dans l'étape d1), peut être effectuée par toute méthode appropriée. A titre d'exemples non limitatifs, les méthodes de détection suivantes peuvent être utilisées :
- électrophorèse sur gel de polyacrylamide (SDS-PAGE) suivie de Western blot ;
- immunoessai, en particulier ELISA direct, indirect, de type sandwich, utilisant des anticorps dirigés contre la protéine prion, de préférence spécifiques du conformère pathogène ;
- essais radioactifs ou fluorescents : l'utilisation de conformère non pathogène marqué (radioactivement avec ³H, ¹⁴C, ³⁵S, ¹²⁵I, etc. ou de manière fluorescente, avec tout fluorophore approprié) pour l'amplification cyclique permet d'obtenir, par transition conformationelle, du conformère pathogène PrP^{SC} néoformé marqué qui peut être détecté et quantifié ;
- utilisation de puces à protéines, capteurs vélocimétriques, biocapteurs électrochimiques, etc.

A titre d'illustration, la détection de la protéine PrP^{SC} peut être effectuée comme décrit dans l'exemple 1 par séparation SDS-PAGE et immunobuvardage à l'aide d'anticorps monoclonaux anti-PrP (par exemple l'anticorps monoclonal 3F4 anti-PrP humaine ou l'anticorps monoclonal 6D11 anti-PrP ovine).

La détection de la présence dudit conformère pathogène PrP^{SC} dans la préparation est indicative de la présence du conformère pathogène PrP^{SC} dans l'échantillon analysé.

Lorsque le procédé selon l'invention est mis en oeuvre sur un échantillon biologique d'un sujet, la détection de la présence dudit conformère pathogène à l'étape d) est indicative d'une maladie à prion ou encéphalopathie spongiforme transmissible (EST) chez le sujet. Le procédé constitue alors un procédé de diagnostic in vitro d'une maladie EST. Si l'échantillon est un prélèvement, par exemple de sang, de plasma ou d'organe , la détection de la présence dudit conformère pathogène PrP^{SC} à l'étape d) indique alors que le prélèvement de sang ou plasma est impropre à la transfusion, ou l'organe impropre à la transplantation, car potentiellement infectieux en tant que vecteur d'une EST.

Lorsque le procédé selon l'invention est mis en oeuvre sur un échantillon qui est un fluide ou solide alimentaire, la détection de la présence dudit conformère pathogène PrP^{SC} à l'étape d) est indicative d'un échantillon contaminé par le conformère pathogène PrP^{SC}, impropre à la consommation car potentiellement infectieux en tant que vecteur d'une EST.

L'étape d) peut comprendre la quantification dudit conformère pathogène PrP^{SC}.

### Kit de détection d'un conformère pathogène de la protéine prion

L'invention concerne également l'utilisation d'un kit pour la mise en oeuvre du procédé selon l'invention.

Un tel kit peut comprendre :
- des nanobilles, de préférence paramagnétiques ;
- du plasminogène, de préférence humain
- éventuellement un autre type de ligand du conformère pathogène de la protéine prion ;
- une quantité connue du conformère non pathogène PrP^{C} ;
- et éventuellement des instructions pour la mise en oeuvre d'un procédé tel que décrit ci-dessus.

De préférence, ledit kit comprend des nanobilles déjà recouvertes de plasminogène et éventuellement dudit au moins un type de ligand du conformère pathogène PrP^{C}.

Le kit peut comprendre en outre au moins un moyen de désagrégation, comme par exemple au moins un solvant ou un appareil de désagrégation physique tel qu'un sonicateur un laser, etc..

Le kit peut comprendre en outre au moins un moyen de détection tel que un ou plusieurs anticorps dirigé(s) contre la protéine prion, de préférence au moins un anticorps spécifique du conformère pathogène.

Les instructions contenues dans le kit peuvent préciser en particulier comment mettre en oeuvre la succession des étapes de capture du conformère pathogène, d'amplification cyclique des protéines mal repliées et de détection.

### Procédé de décontamination d'un échantillon biologique

Les nanobilles recouvertes de plasminogène peuvent également être utilisées pour capturer les protéines prions PrP^{SC} présentes dans un échantillon biologique de manière à le décontaminer en le débarrassant des protéines PrP^{SC} qu'il contient.

La demande décrit ainsi également un procédé de décontamination d'un échantillon biologique susceptible de contenir du conformère pathogène de la protéine prion (PrP^{SC}), ledit procédé comprenant les étapes consistant à :
a) mettre en contact des nanobilles, de préférence paramagnétiques, recouvertes de plasminogène avec un échantillon;
b) séparer lesdites nanobilles paramagnétiques recouvertes de plasminogène de l'échantillon ;
c) récolter l'échantillon ainsi décontaminé.

Dans l'étape a) de ce procédé, les nanobilles recouvertes de plasminogène sont mises en contact avec l'échantillon à analyser et incubées au contact de l'échantillon pendant une durée suffisante et dans des conditions permettant la liaison au plasminogène de tout les conformères pathogènes PrP^{SC} présents dans l'échantillon.

La durée et les conditions d'incubation peuvent être variables en fonction de la nature de l'échantillon.

L'incubation peut être effectuée à n'importe quelle température, par exemple au froid (4-10°C), à température ambiante ou à 37°C. Elle est préférentiellement mise en oeuvre à 37°C. L'incubation peut être effectuée avec agitation (par exemple de 1 rpm à 1400 rpm) ou sans agitation. L'incubation peut être effectuée pendant une durée de 1 minute à 5 jours, de préférence pendant 10 minute à 24 heures, de préférence 30 minutes à 8 heures, de préférence 1 à 4 heures, de préférence encore de 2 heure à 3 heures. La durée d'incubation est susceptible d'être ajustée en fonction des quantités respectives de conformère pathogène de la protéine prion dans l'échantillon et de plasminogène fixé sur le support solide.

Les nanobilles recouvertes de plasminogène peuvent être séparées de l'échantillon (étape b)) par exemple en récoltant les nanobilles par aimantation si les billes sont paramagnétiques ou par centrifugation.

La décontamination de l'échantillon biologique ainsi traité peut être contrôlée en mettant en oeuvre un procédé in vitro de détection d'un conformère pathogène de la protéine prion (PrP^{SC}) dans un échantillon tel que défini plus haut, de manière à s'assurer de l'absence de conformère pathogène de la protéine prion dans l'échantillon.

Les définitions et conditions décrites en relation avec le procédé de détection d'un conformère pathogène de la protéine prion selon l'invention, sont applicables également au kit de détection d'un conformère pathogène de la protéine prion et procédé de décontamination d'un échantillon définis ci-dessus.

L'invention sera décrite plus en détail au vu des exemples suivants.

### EXEMPLES

### Exemple 1 : Matériels et méthodes

### Préparation des échantillons

Du sang complet de volontaires humains a été prélevé dans des tubes de collecte contenant de l'EDTA (Etablissement Français du Sang - Pyrénées, Méditerranée) après avoir obtenu le consentement éclairé écrit de chaque donneur de sang, en accord avec la loi française (Code de la santé publique, article L. 1243-3). Le plasma a ensuite été isolé et récolté après centrifugation à 1500 g pendant 15 minutes à température ambiante.

Du sang complet a été récolté de quatre moutons sains et de quatre moutons naturellement infectés par la tremblante, au stade terminal de la maladie (INRA/Ecole Nationale Vétérinaire de Toulouse - France). Les moutons exprimaient l'allèle V136R154Q171 de la PrP ovine. Les globules blancs sanguins de moutons (SWBC) ont été préparés à partir de la couche leucocytaire obtenue après centrifugation à 2000 g pendant quinze minutes à température ambiante. Les globules rouges résiduels ont été éliminés de la couche leucocytaire par lyse dans NH₄Cl 155 mM, KHCO₃10 mM, EDTA 1 mM (pH 8) pendant 20 minutes sur la glace, suivie d'une centrifugation à 1500 g pendant 10 minutes à 4 °C. Les SWBC ont ensuite été lavés avec du PBS pH 7,4 et des aliquotes de 10⁷ SWBC clarifiés par le PBS ont été stockés à -80°C.

Des plasmas dopés ont été préparés comme suit : 500 µl de plasma de donneurs humains sains ont été dopés avec des dilutions en série au dixième (de 10⁻³ à 10⁻¹¹) soit d'homogénats de cerveau infecté par la variante de la maladie de Creutzfeldt-Jacob (vMCJ), soit d'homogénats de cerveau infecté avec la tremblante (souche 127S) provenant de souris transgéniques ovines (lignée tg338). Les homogénats de cerveau infecté (IBH) ont été préparés à 10% (poids/volume) dans du glucose 5% (c'est-à-dire une dilution 10⁻¹).

### Capture de la protéine prion

### Recouvrement de billes magnétiques avec du plasminogène humain (Plg)

Selon les études publiées par Fischer et al. (Nature, 2000, 408 (6811) : 479-483), du plasminogène humain a été utilisé en tant que ligand efficace pour la capture des protéines prions. Des billes caboxyliques nanomagnétiques (Ademtech France) ont été recouvertes et stockées conformément aux instructions du fabricant. Brièvement, les billes ont été recouvertes de plasminogène humain (Fluka Sigma-Aldrich) par agitation pendant 2 heures à 37°C. La quantité de ligand optimale à utiliser a été évaluée en testant trois concentrations de plasminogène : 10, 20 et 30 µg/mg de billes, 10 µg/mg de billes étant la concentration la plus faible recommandée par le fabricant. Après une étape de blocage à 37°C avec une solution d'albumine à 0,5 mg/ml, les billes ont été stockées à 4°C dans la solution de stockage Ademtech.

### Capture de la protéine prion par les nanobilles recouvertes de plasminogène

Les plasmas dopés (500 µl) ont été mélangés volume à volume avec un tampon de lyse/liaison (tampon LB: PBS 2X, 6% NP40, 6% Tween 20) avant incubation avec les billes recouvertes de plasminogène à température ambiante pendant 90 minutes. La quantité de billes à utiliser pour chaque échantillon a été évaluée en testant différentes conditions (2,5, 5, 10, 20, 30, 40, 60 et 90 µl de billes plg à 1%). Après une étape de lavage avec du PBS, les billes magnétiques ont été isolées pour amplification PMCA ultérieure.

Pour la capture de PrP^{EST} à partir des SWBC, les cellules ont été d'abord incubées sur la glace pendant 30 minutes avec 500 µl de tampon LB. Après centrifugation à 1000 g à 4°C pendant 2 minutes, le surnageant a été récolté et mélangé avec 500 µl de tampon LB avant incubation avec les nanobilles recouvertes de plasminogène comme décrit ci-dessus.

### PMCA (Protein Misfolding Amplification Assay, test d'amplification de protéines mal repliées)

### Préparation des substrats PMCA :

Les homogénats de cerveau normal (NBH) de souris transgéniques soit pour la PrP humaine (allèle M¹²⁹, lignée tg650) (Beringue et al., PLoS One 2008, 3 (1) :e1419) soit pour la PrP ovine (allèle VRQ, lignée tg338) (Laude et al., C. R. Biol. 2002, 325 (1):49-57) ont été utilisés en tant que substrat PrP^{C} pour la PMCA. Les cerveaux ont été collectés à partir d'animaux euthanasiés avec du CO₂ et perfusés avec du PBS contenant 5 mM d'EDTA. Les cerveaux ont été rincés dans du PBS froid et immédiatement congelés sur carbo-glace avant stockage à long terme à -80°C. Les cerveaux ont ensuite été homogénéisés à une concentration de 10 % (poids/volume) avec le tampon de lyse/conversion (tampon CB) composé de NaCl 150 mM, Triton X-100 1%, cocktail d'inhibiteurs de protéases (Roche) dans du PBS (pH 7,2). Les homogénats ont été centrifugés à 2000 g pendant 20 secondes pour retirer le gros des matériaux de cerveau. Le surnageant a été congelé à -80° C en aliquotes à usage unique jusqu'à l'utilisation.

### PMCA

La protéine prion capturée a été mélangée avec 90 µl de NBH à 10%. Ensuite, une amplification automatique par PMCA a été mise en oeuvre en utilisant le Misonix 4000 (Misonix, NY, USA). Chaque cycle était composé d'une étape d'incubation (37 °C) et d'une étape de sonication. Au préalable, les paramètres de PMCA suivants avaient été optimisés : durée d'incubation par cycle (30 et 60 minutes, ou encore 90 minutes pour le premier cycle), durée de sonication par cycle (20 et 40 secondes), niveau de puissance (60, 70, 80 %) et nombre de cycles de PMCA (50, 80, 100) par tour. Suite au premier tour de PMCA, le produit de réaction est dilué dans du substrat frais (1/10) et la PMCA est répétée. Cette technique est connue en tant que PMCA en série (sPMCA). Le produit de ce deuxième tour de PMCA peut encore être dilué et soumis à PMCA, et ainsi de suite, permettant une amplification indéfinie de PrP^{EST}.

### Digestion par la protéinase K (PK)

Les produits amplifiés (échantillons de 18 µl) ont été digérés avec PK à 200 µg/ml pendant une heure à 45°C. La réaction a été stoppée par addition de phénylméthyle sulfonide fluorure 5 mM. Les échantillons ont ensuite été dénaturés à 100°C dans un tampon pour échantillon SDS-PAGE (SDS 2%, Tris/HCl 100 mM pH 7,4 et bleu de bromophénol 0,5 %) et stockés à -80°C.

### SDS-PAGE et immunoblots

Les échantillons protéiques ont été mis à migrer sur des gels NUPAGE 12% (Invitrogen, Cergy-Pontoise, France) à 200 volts, et électrotransférés sur des membranes PVDF (Millipore, Molsheim, France). Après blocage avec du lait écrémé 5% dans un tampon TBS-Tween 20 0,05 %, les transferts ont été sondés avec des anticorps monoclonaux anti-PrP, 3F4 MAb ou 6D11 MAb (Signet/Proteogenix, Illkirch, France) pour la détection de la protéine prion humaine et ovine, respectivement.

Les transferts ont été développés en utilisant un anticorps secondaire anti-IgG de souris lié à la peroxydase et le substrat chimioluminescent Luminol (ECL reagent, Amersham Bioscience, France).

### Matériels de l'Institut national pour les étalons et contrôles biologiques (NIBSC)

Le NIBSC a rendu disponible une série de réactifs de référence préparés à partir de spécimens de cerveau provenant d'humains autopsiés (www.nibsc.ac.uk/cjd/brainsamples.html). Les réactifs de référence de cerveau ont été obtenus à partir du Centre Ressource CJD sous la forme d'homogénats à 10% (poids/volume) dans du saccharose 0,25 M.

Deux panels ont été testés. Le panel 1 était composé de 20 échantillons de plasma négatif incluant 10 plasmas choisis au hasard distribués en duplicats. Le panel 2 était un panel en aveugle composé de duplicats de dilution de cerveau vCJD dans du plasma normal, et de contrôles négatifs incluant du cerveau normal additionné dans du plasma et du plasma négatif seul (4 séries de 24 tubes numérotés de A à X).

### Exemple 2: Optimisation de la technologie capture basée sur le plasminogène/sPMCA en utilisant du matériel de cerveau infecté par la tremblante

Les paramètres de PMCA ont été optimisés en utilisant des homogénats de cerveau normal et infecté par la tremblante provenant de souris transgéniques pour la PrP ovine (allèle VRQ, lignée tg338) pour la réaction PMCA. La PMCA a été d'abord évaluée en croisant les paramètres de nombre de cycles, incubation, sonication et puissance du Misonix. Un tour de 80 cycles de PMCA (incubation de 30 minutes, sonication de 20 secondes, puissance 80 %) a permis la détection de PrP^{EST} dans la dilution IBH 10⁻⁸ par immunoblot (n=3 à partir de 2 IBH différents). Le niveau de signal observé était similaire à celui obtenu pour la dilution 10⁻³ non amplifiée, indiquant un niveau d'amplification de 5 log par le premier tour de PMCA. Lorsque les échantillons amplifiés ont été dilués 10 fois dans du NBH frais et soumis à un deuxième tour de 80 cycles de PMCA, un signal de détection spécifique de PrP^{EST} a été détecté de manière reproductible jusqu'à la dilution 10⁻¹⁰ (n=3 à partir de 2 IBH différents), indiquant une amplification de 7 log. Aucun signal n'a été détecté avec les cerveaux non infectés amplifiés de manière similaire (n=3 à partir de 2 IBH différents).

La PMCA étant optimisée, les paramètres de capture de la PrP^{EST} par les nanobilles recouvertes de plasminogène ont ensuite été optimisés (concentration de plasminogène et volume de billes). Une dilution de IBH avec la tremblante à 10⁻⁴ a été diluée dans du plasma humain (500 µl) et mélangée avec différents volumes de billes avec différentes concentrations de plasminogène avant amplification PMCA. Le signal le plus fort de PrP^{EST} a été observé lorsqu'ont été utilisés la concentration la plus faible de plasminogène, à 10 µg plasminogène/mg de billes, et le volume le plus faible de billes, 10 µl de billes/500 µl de plasma dopé.

En utilisant ces conditions, PrP^{EST} a pu être détectée dans des dilutions d'IBH à 10⁻⁶ additionnées dans 500 µl de plasma humain. Le signal obtenu est apparu similaire à celui obtenu lorsqu'une dilution d'IBH 10⁻⁶ a été utilisée directement pour ensemencer le substrat PMCA, suggérant que l'étape de capture par les nanobilles recouvertes de plasminogène était efficace. Les deux contrôles négatifs (500 µl de plasma humain mélangés avec des nanobilles recouvertes de plasminogène avant PMCA) sont restés négatifs.

### Exemple 3 : validation de l'essai combiné nanobilles recouvertes de plasminogène/sPMCA sur un panel de sang de moutons sains et infectés

Il a ensuite été vérifié que les conditions expérimentales utilisées permettaient de détecter spécifiquement PrP^{EST} dans des SWBC infectés par la tremblante. Après un tour de PMCA (80 cycles), aucun signal spécifique de PrP^{EST} n'a été détecté par immunoblot dans les 4 échantillons infectés de SWBC. Après un deuxième tour, un des 4 SWBC infectés a montré un signal positif. Après un troisième tour de PMCA, un signal PrP^{EST} fort a été détecté dans la totalité des 4 SWBC infectés, alors que dans les échantillons SWBC provenant de moutons sains, aucun signal n'a été détecté. La détection des SWBC de moutons infectés par la tremblante et négatifs a été reproduite 3 fois.

Par comparaison, la mise en oeuvre de la méthode sPMCA sur des SWBC infectés par la tremblante, sans étape préalable de capture, n'a pas permis de détecter la protéine PrP^{EST}.

### Exemple 4: Evaluation et validation du test nanobilles recouvertes de plasminogène/sPMCA sur des échantillons humains

Des optimisations similaires de la PMCA ont été mises en oeuvre en utilisant les homogénats de cerveau humain vCJD et des homogénats de cerveau normal provenant de souris transgéniques pour la PrP humaine (allèle M¹²⁹, lignée tg650), comme décrit pour la PMCA ovine, et les paramètres donnant l'amplification optimale pour la PMCA humaine ont été identifiés comme étant exactement les mêmes (80 cycles, 30 minutes d'incubation, 20 secondes de sonication, puissance 80 %).

Après capture de la PrP^{EST} dans des plasmas humains dopés par addition de dilutions au 1/10 d'IBH vCJD (10⁻⁵ à 10⁻⁹), un premier tour de PMCA a permis la détection de la protéine dans les dilutions IBH à 10⁻⁶. Ceci indiquait une amplification d'environ 3 log comparée au signal obtenu à partir de la dilution 10⁻³ non amplifiée.

Après une dilution au 1/10 des échantillons amplifiés avec des NBH frais, un deuxième et un troisième tour de 80 cycles de PMCA ont été réalisés. Ceci a permis la détection d'un signal spécifique de PrP^{EST} jusqu'aux dilutions 10⁻⁷ et 10⁻⁹ respectivement, indiquant un facteur d'amplification de 4 et 6 log. La sensibilité du test a été confirmée en répétant ce test à partir de trois IBH différents, l'un d'eux étant le panel NIBSC "blue capped brain tissue vCJD codon 129M/M" (Ref NHBY0/0003).

Ce test combinant la capture basée sur le plasminogène du prion dans des échantillons de sang humain et l'amplification PMCA a ensuite été évalué en testant des panels fournis par le NIBSC. Tous les échantillons du panel 1 (échantillons négatifs) ont été testés négatifs (20/20) conduisant à une spécificité de 100 %.

Les résultats de l'analyse à l'aveugle des 80 échantillons du panel 2 ont été analysés par le comité NIBSC et une sensibilité d'une dilution de 10⁻⁵ pour les homogénats de cerveau vMCJ additionnés dans le plasma a été obtenue (tableau 1). Les 32 échantillons contrôle du panel 2 ont tous été testés négatifs et ont confirmé la spécificité de 100 % (total 52/52).

**Tableau 1 : Evaluation du test de détection de PrP^{EST} sur un panel humain à l'aveugle (NIBSC)**

| Matériel biologique | Dilutions | Positif |
|---|---|---|
| Plasma dopé de cerveau vMCJ | 10⁻² | 4/4 |
| | 10⁻³ | 4/4 |
| | 10⁻⁴ | 4/4 |
| | 10⁻⁵ | 3/8 |
| | 10⁻⁶ | 1/4 |
| | 10⁻⁷ | 0/4 |
| | 10⁻⁸ | 0/8 |
| Plasma dopé de rate vMCJ | 10⁻¹ | 4/4 |
| | 10⁻² | 0/4 |
| | 10⁻³ | 0/8 |
| | 10⁻⁴ | 0/4 |
| | 10⁻⁵ | 0/4 |
| Plasma dopé de cerveau contrôle | 10⁻² | 0/4 |
| | 10⁻³ | 0/4 |
| | 10⁻⁴ | 0/4 |
| Plasma dopé de rate contrôle | 10⁻¹ | 0/4 |
| | 10⁻² | 0/4 |
| Plasma contrôle | -- | 0/16 |
| Total positif | | 20/96 |

## Revendications

1. Procédé in vitro de détection d'un conformère pathogène de la protéine prion (PrP^{SC}) dans un échantillon, ledit procédé comprenant les étapes consistant à :
a) mettre en contact des nanobilles recouvertes de plasminogène avec un échantillon;
b) séparer lesdites nanobilles recouvertes de plasminogène portant la PrPsc éventuellement présente, de l'échantillon ;
c1) mettre en contact lesdites nanobilles recouvertes de plasminogène portant la PrP^{SC} éventuellement présente, obtenues à l'étape b) avec une préparation comprenant du conformère non pathogène de la protéine prion (PrP^{C}) ;
c2) désagréger les agrégats éventuellement formés durant l'étape c1) ;
d) détecter la présence de PrP^{SC} dans la préparation, la présence de PrP^{SC} dans la préparation étant indicative de la présence de PrP^{SC} dans l'échantillon ;
les étapes c1) et c2) formant un cycle qui est répété au moins deux fois avant de mettre en oeuvre l'étape d).

2. Procédé in vitro selon la revendication 1, dans lequel l'étape d) est précédée d'une étape d1) de destruction ou élimination de la protéine PrP^{C} présente dans la préparation à l'issue de l'étape c2).

3. Procédé in vitro selon la revendication 1 ou 2, dans lequel les nanobilles sont des nanobilles paramagnétiques recouvertes de plasminogène à raison de 10 à 30 µg de plasminogène/mg de nanobilles.

4. Procédé in vitro selon l'une quelconque des revendications 1 à 3, dans lequel les nanobilles recouvertes de plasminogène sont mises en contact avec l'échantillon à l'étape a) à un ratio de : 2,5 à 90 µl de suspension de nanobilles à 1% (poids/volume) pour 50 à 500 µl d'échantillon.

5. Procédé in vitro selon l'une quelconque des revendications 1 à 4, dans lequel l'étape a) est effectuée avec agitation et la désagrégation de l'étape c2) est effectuée par sonication.

6. Procédé in vitro selon l'une quelconque des revendications 1 à 5, qui comprend les étapes consistant à :
a) mettre en contact des nanobilles paramagnétiques recouvertes de plasminogène avec un échantillon, à raison de 10 à 30 µg de plasminogène/mg de nanobilles paramagnétiques ;
b) séparer lesdites nanobilles paramagnétiques recouvertes de plasminogène portant la PrP^{SC} éventuellement présente, de l'échantillon ;
c1) mettre en contact, pendant 20 minutes à 2 heures, lesdites nanobilles paramagnétiques recouvertes de plasminogène portant la PrP^{SC} éventuellement présente, obtenues à l'étape b) avec une préparation comprenant du conformère non pathogène de la protéine prion (PrP^{C});
c2) désagréger par sonication les agrégats éventuellement formés durant l'étape c1) pendant 10 secondes à 1 minute ;
d) détecter la présence de PrP^{SC} dans la préparation, la présence de PrP^{SC} dans la préparation étant indicative de la présence de PrP^{SC} dans l'échantillon ;
les étapes c1) et c2) formant un cycle qui est mis en oeuvre de 50 à 350 fois avant de mettre en oeuvre l'étape d).

7. Procédé in vitro l'une quelconque des revendications 1 à 6, qui comprend :
- un premier cycle d'amplification dans lequel l'étape c1) est mise en oeuvre pendant 60 à 120 minutes et l'étape c2) est mise en oeuvre par sonication pendant 10 secondes à 1 minutes, puis
- un ou plusieurs cycles d'amplification dans lesquels l'étape c1) est mise en oeuvre pendant 30 à 60 minutes.

8. Procédé in vitro selon la revendication 7, qui comprend :
- un premier cycle d'amplification pendant lequel l'étape c1) est mise en oeuvre pendant 80 à 100 minutes, et l'étape c2) est mise en oeuvre par sonication pendant 20 à 40 secondes, puis
- 49 à 99 cycles d'amplification pendant lesquels l'étape c1) est mise en oeuvre pendant 30 à 60 minutes, et l'étape c2) est mise en oeuvre par sonication pendant 20 à 40 secondes.

9. Procédé in vitro selon l'une quelconque des revendications 1 à 8, dans lequel ledit échantillon est un échantillon biologique d'un sujet et la détection de la présence de PrP^{SC} à l'étape d) est indicative d'une maladie encéphalopathie spongiforme transmissible chez le sujet.

10. Procédé in vitro selon la revendication 9, dans lequel l'encéphalopathie spongiforme transmissible est la maladie de Creutzfeld Jacob ou sa variante.

11. Procédé in vitro selon l'une quelconque des revendications 1 à 10, dans lequel ledit échantillon est un fluide ou solide alimentaire et la détection de la présence de PrP^{SC} à l'étape d) est indicative d'un fluide ou solide alimentaire contaminé par le conformère pathogène PrP^{SC}.

12. Procédé in vitro selon l'une quelconque des revendications 1 à 11, dans lequel ledit échantillon est, ou provient de, un prélèvement de sang, de plasma ou d'organe et la détection de la présence de PrP^{SC} à l'étape d) est indicative d'un prélèvement de sang ou plasma impropre à la transfusion, ou d'un organe impropre à la transplantation.

13. Procédé in vitro selon l'une quelconque des revendications 1 à 12, dans lequel l'étape d) comprend la quantification dudit conformère pathogène.

14. Utilisation d'un kit comprenant :
- des nanobilles paramagnétiques;
- du plasminogène ;
- une quantité connue du conformère non pathogène de la protéine prion PrP^{C},
pour la mise en oeuvre d'un procédé selon l'une quelconque des revendications 1 à 13.

15. Utilisation d'un kit selon la revendication 14, comprenant en outre au moins un autre ligand d'un conformère pathogène de la protéine prion PrP^{SC}.

## Patentansprüche

1. Ein in vitro Verfahren zum Nachweis eines pathogenen Konformers eines Prionenproteins (PrP^{SC}) in einer Probe, das Verfahren umfassend die Schritte bestehend aus:
a) In-Kontakt-bringen von mit Plasminogen beschichteten Nanopartikeln mit einer Probe;
b) Abtrennen der mit Plasminogen beschichteten Nanopartikel, die eventuell vorhandenes PrP^{SC} tragen, von der Probe;
c1) In-Kontakt-bringen der mit Plasminogen beschichteten Nanopartikel, die eventuell vorhandenes PRP^{SC} tragen, erhalten in Schritt b), mit einem Präparat umfassend das nicht-pathogene Konformer des Prionenproteins (PrP^{C});
c2) Auflösen von eventuell während Schritt c1) gebildeten Aggregaten;
d) Nachweisen des Vorhandenseins von PrP^{SC} in dem Präparat, wobei das Vorhandensein von PrP^{SC} in dem Präparat das Vorhandensein von PrP^{SC} in der Probe anzeigt;
wobei die Schritte c1) und c2) einen Zyklus bilden, der mindestens zweimal vor dem Durchführen von Schritt d) wiederholt wird.

2. Das in vitro Verfahren gemäß Anspruch 1, in welchem dem Schritt d) ein Schritt d1) der Zerstörung oder Beseitigung des Proteins PrP^{C}, welches in dem Präparat vorhanden ist, nach Beendigung von Schritt c2) vorausgeht.

3. Das in vitro Verfahren gemäß Anspruch 1 oder 2, in welchem die Nanopartikel paramagnetische Nanopartikel sind, welche mit Plasminogen im Bereich von 10 bis 30 µg Plasminogen/mg Nanopartikel beschichtet sind.

4. Das in vitro Verfahren gemäß einem der Ansprüche 1 bis 3, in welchem die mit Plasminogen beschichteten Nanopartikel in Schritt a) mit einer Probe in einem Verhältnis von 2,5 bis 90 µl einer 1%-igen Suspension von Nanopartikeln (Gewicht/Volumen) für 50 bis 500 µl der Probe in Kontakt gebracht werden.

5. Das in vitro Verfahren gemäß einem der Ansprüche 1 bis 4, in welchem der Schritt a) unter Schütteln durchgeführt wird und die Auflösung in Schritts c2) mit Ultraschall durchgeführt wird.

6. Das in vitro Verfahren gemäß einem der Ansprüche 1 bis 5, welches die folgenden Schritte umfasst, die bestehen aus:
a) In-Kontakt-bringen der mit Plasminogen beschichteten paramagnetischen Nanopartikel mit einer Probe im Bereich von 10 bis 30 µg Plasminogen/mg paramagnetischer Nanopartikel;
b) Abtrennen der mit Plasminogen beschichteten paramagnetischen Nanopartikel, die eventuell vorhandenes PrP^{SC} tragen, von der Probe;
c1) In-Kontakt-bringen, in einem Zeitraum von 20 Minuten bis 2 Stunden, der mit Plasminogen beschichteten paramagnetischen Nanopartikel, die eventuell vorhandenes PrP^{SC} tragen, erhalten in Schritt d), mit einem Präparat umfassend ein nichtpathogenes Konformer des Prionenproteins (PrP^{C});
c2) Auflösen eventuell in Schritt c1) gebildeter Aggregate mittels Ultraschall über einen Zeitraum von 10 Sekunden bis 1 Minute;
d) Nachweisen des Vorhandenseins von PrP^{SC} in dem Präparat, wobei das Vorhandensein von PrP^{SC} in dem Präparat das Vorhandensein von PrP^{SC} in der Probe anzeigt;
wobei die Schritte c1) und c2) eine Zyklus bilden, der 50 bis 350 mal vor dem Durchführen von Schritt d) durchgeführt wird.

7. Das in vitro Verfahren gemäß einem der Ansprüche 1 bis 6, welches umfasst:
- einen ersten Amplifizierungszyklus, in dem Schritt c1) über einen Zeitraum von 60 bis 120 Minuten durchgeführt wird und Schritt c2) mittels Ultraschall über einen Zeitraum von 10 Sekunden bis 1 Minute durchgeführt wird, dann
- einen oder mehrere Amplifikationszyklen, in denen Schritt c1) über einen Zeitraum von 30 bis 60 Minuten durchgeführt wird.

8. Das in vitro Verfahren gemäß Anspruch 7, welches umfasst:
- einen ersten Amplifikationszyklus, in dessen Verlauf Schritt c1) über einen Zeitraum von 80 bis 100 Minuten durchgeführt wird und Schritt c2) mittels Ultraschall über einen Zeitraum von 20 bis 40 Sekunden durchgeführt wird, dann
- 49 bis 99 Amplifikationszyklen, in deren Verlauf Schritt c1) über einen Zeitraum von 30 bis 60 Minuten durchgeführt wird und Schritt c2) mittels Ultraschall über einen Zeitraum von 20 bis 40 Sekunden durchgeführt wird.

9. Das in vitro Verfahren gemäß einem der Ansprüche 1 bis 8, in welchem die Probe eine biologische Probe eines Subjekts ist und der Nachweis des Vorhandenseins von PrP^{SC} in Schritt d) eine übertragbare, enzephalopathische spongiforme Krankheit bei dem Subjekt anzeigt.

10. Das in vitro Verfahren nach Anspruch 9, in welchen die übertragbare, enzephalopathische spongiforme Krankheit die Creutzfeld-Jacob-Krankheit oder eine Variante davon ist.

11. Das in vitro Verfahren gemäß einem der Ansprüche 1 bis 10, in welchem die Probe ein flüssiges oder festes Nahrungsmittel ist und der Nachweis des Vorhandenseins von PrP^{SC} in Schritt d) ein mit dem pathogenen PrP^{SC} Konformer kontaminiertes flüssiges oder festes Nahrungsmittel anzeigt.

12. Das in vitro Verfahren gemäß einem der Ansprüche 1 bis 11, in welchem die Probe eine Blut-, Plasma oder Organprobe ist oder davon abstammt, und der Nachweis des Vorhandenseins von PrP^{SC} in Schritt d) eine zur Transfusion ungeeignete Blutprobe oder Plasmaprobe oder ein zur Transplantation ungeeignetes Organ anzeigt.

13. Das in vitro Verfahren nach einem der Ansprüche 1 bis 12, in welchem Schritt d) die Quantifizierung des erwähnten pathogenen Konformers umfasst.

14. Verwendung eines Kits umfassend:
- paramagnetische Nanopartikel;
- Plasminogen;
- eine bekannte Menge des nicht-pathogenen Konformers des Prionenproteins PrP^{C}
für die Durchführung eines Verfahrens gemäß einem der Ansprüche 1 bis 13.

15. Verwendung eines Kits gemäß Anspruch 14, welches des Weiteren mindestens einen anderen Liganden des pathogenen Konformers des Prionenproteins PrP^{SC} umfasst.

## Claims

1. An *in vitro* method for detection of a pathogenic conformational isomer of the prion protein (PrP^{SC}) in a sample, said method comprising the steps consisting of:
a) putting nanobeads covered with plasminogen into contact with a sample;
b) separating said nanobeads covered with plasminogen bearing the possibly present PrP^{SC}, from the sample;
c1) putting said nanobeads covered with plasminogen bearing the possibly present PrP^{SC} obtained in step b) in contact with a preparation comprising some non-pathogenic conformational isomer of the prion protein (PrP^{C});
c2) disaggregating the aggregates possibly formed during step c1);
d) detecting the presence of PrP^{SC} in the preparation, the presence of PrP^{SC} in the preparation being indicative of the presence of PrP^{SC} in the sample;
the steps c1) and c2) forming a cycle which is repeated at least twice before applying step d).

2. The *in vitro* method according to claim 1, wherein step d) is preceded with a step d1) for destroying or removing the protein PrP^{C} present in the preparation at the end of step c2).

3. The *in vitro* method according to claim 1 or 2, wherein the nanobeads are paramagnetic nanobeads covered with plasminogen
in an amount from 10 to 30 µg of plasminogen/mg of nanobeads.

4. The *in vitro* method according any one of claims 1 to 3, wherein the nanobeads covered with plasminogen are put into contact with the sample in step a) at a ratio from 2.5 to 90 µl of 1% (weight/volume) nanobead suspension for 50 to 500 µl of sample.

5. The *in vitro* method according to any one of claims 1 to 4, wherein step a) is carried out with stirring and
disaggregation of step c2) is carried out by sonication.

6. The *in vitro* method according to any one of claims 1 to 5, which comprises the steps consisting of:
a) putting the paramagnetic nanobeads covered with plasminogen into contact with a sample, in an amount from 10 to 30 µg of plasminogen/mg of paramagnetic nanobeads;
b) separating said paramagnetic nanobeads covered with plasminogen bearing the possibly present PrP^{SC}, from the sample;
c1) putting said paramagnetic nanobeads covered with plasminogen bearing the possibly present PrP^{SC} obtained in step b) into contact for 20 minutes to 2 hours with a preparation comprising some non-pathogenic conformational isomer of the prion protein (PrP^{C});
c2) disaggregating by sonication the aggregates possibly formed during step c1) for 10 seconds to 1 minute;
d) detecting the presence of PrP^{SC} in the preparation, the presence of PrP^{SC} in the preparation being indicative of the presence of PrP^{SC} in the sample;
steps c1) and c2) forming a cycle which is applied from 50 to 350 times before applying step d).

7. The *in vitro* method according to any one of claims 1 to 6, which comprises:
- a first amplification cycle in which step c1) is applied for 60 to 120 minutes and step c2) is applied by sonication for 10 seconds to 1 minute, and then
- one or several amplification cycles in which step c1) is applied for 30 to 60 minutes.

8. The *in vitro* method according to claim 7 which comprises:
- a first amplification cycle during which step c1) is applied for 80 to 100 minutes, and step c2) is applied by sonication for 20 to 40 seconds, and then
- 49 to 99 amplification cycles during which step c1) is applied for 30 to 60 minutes, and step c2) is applied by sonication for 20 to 40 seconds.

9. The *in vitro* method according to any one of claims 1 to 8, wherein said sample is a biological sample from a subject and the detection of the presence of PrP^{SC} in step d) is indicative of a transmissible spongiform encephalopathy disease in the subject.

10. The *in vitro* method according to claim 9, wherein the transmissible spongiform encephalopathy is Creutzfeld Jacob's disease or its variant.

11. The *in vitro* method according to any one of claims 1 to 10, wherein said sample is a food fluid or solid and the detection of the presence of PrP^{SC} in step d) is indicative of a food fluid or solid contaminated by the pathogenic conformational isomer PrP^{SC}.

12. The *in vitro* method according to any one of claims 1 to 11, in which said sample is, or stems from a sample taken from blood, plasma or from an organ and the detection of the presence of PrP^{SC} in step d) is indicative of a sample taken from blood or plasma unsuitable for transfusion, or from an organ unsuitable for transplantation.

13. The *in vitro* method according to any one of claims 1 to 12, wherein step d) comprises the quantification of said pathogenic conformational isomer.

14. Use of a kit comprising:
- paramagnetic nanobeads;
- plasminogen;
- a known amount of the non-pathogenic conformational isomer of the prion protein PrP^{C};
for applying a method according to any one of claims 1 to 13.

15. Use of a kit according to claim 14, further comprising at least one other ligand of a pathogenic conformational isomer of the prion protein PrP^{SC}
